# EUROPEAN PATENT SPECIFICATION

(11) **EP 4 071 474 B1**
(45) Date of publication and mention of the grant of the patent: **06.03.2024**
(21) Application number: 21382293.5
(22) Date of filing: 07.04.2021
(51) Int. Cl.: G01N 33/543, G01N 33/58

(54) **MICROFLUIDIC DEVICES AND METHODS**
MIKROFLUIDISCHE VORRICHTUNGEN UND VERFAHREN
DISPOSITIFS ET PROCÉDÉS MICROFLUIDIQUES

(43) Date of publication of application: 12.10.2022
(73) Proprietor: Fundación Tecnalia Research & Innovation, 20009 San Sebastián (ES); Asociación Centro de Investigación Cooperativa en Biomateriales - CIC biomaGUNE, 20014 San Sebastián (ES)
(72) Inventor: PAVLOV, Valery, 20115 ASTIGARRAGA (ES); DIAZ-BUITRAGO, Beatriz, 20009 SAN SEBASTÍAN (ES); BRIZ ICETA, Nerea, 20019 SAN SEBASTIÁN (ES); BIJELIC, Goran, 20009 SAN SEBASTIÁN (ES)
(74) Representative: ZBM Patents - Zea, Barlocci & Markvardsen

(56) References cited:
- WO-A1-2019/134741
- GRINYTE RUTA ET AL: "Microbead QD-ELISA: Microbead ELISA Using Biocatalytic Formation of Quantum Dots for Ultra High Sensitive Optical and Electrochemical Detection", APPLIED MATERIALS & INTERFACES, vol. 8, no. 43, 2 November 2016 (2016-11-02), pages 29252-29260, XP055841743, US ISSN: 1944-8244, DOI: 10.1021/acsami.6b08362 Retrieved from the Internet: URL:https://pubs.acs.org/doi/pdf/10.1021/a csami.6b08362>
- BARROSO JAVIER ET AL: "Specific bioanalytical optical and photoelectrochemical assays for detection of methanol in alcoholic beverages", BIOSENSORS AND BIOELECTRONICS, vol. 101, 13 October 2017 (2017-10-13), pages 116-122, XP085262113, ISSN: 0956-5663, DOI: 10.1016/J.BIOS.2017.10.022
- DÍEZ-BUITRAGO BEATRIZ ET AL: "Development of portable CdS QDs screen-printed carbon electrode platform for electrochemiluminescence measurements and bioanalytical applications", TALANTA, ELSEVIER, AMSTERDAM, NL, vol. 225, 17 December 2020 (2020-12-17), XP086496408, ISSN: 0039-9140, DOI: 10.1016/J.TALANTA.2020.122029 [retrieved on 2020-12-17]

## Description

The present disclosure relates to microfluidic devices. The present disclosure relates to methods of detecting an antigen of a fluid sample using an enzyme linked immunosorbent assay.

### BACKGROUND

Enzyme-linked immunosorbent assay (ELISA) is a plate-based assay technique designed for detecting and quantifying soluble substances such as peptides, proteins, antibodies, antigens, and hormones. In an ELISA, the target antigen is immobilized on a solid surface and then contacted with an enzyme labeled detection antibody. Detection is accomplished by measuring the activity of the enzyme via incubation with the appropriate reagent to produce a measurable product. ELISA is based on a highly specific antibody-antigen interaction.

ELISA is performed on microtiter plates and evaluated with dedicated readers that are bulky and/or expensive. Therefore, ELISA is not suitable for low-cost and portable applications. The procedure demands trained operators, complementary tools and disposables for volume metering, a clean operation environment (i.e. a laboratory setting with dedicated instruments, including plate washers) and a specialized readout system (e.g. optical colorimetric, densitometry or fluorescent detectors). ELISAs are slow (e.g., several hours up to 24 hours) and require large volumes of samples and reagents.

In an ELISA, sample and calibration concentrations are pipetted into functionalized wells (consuming one disposable tip in each operation), incubated for a given time; and after the removal of sample and calibration volumes, washed in multiple refill operations. The procedure is then repeated for labelling the detection antibody.

In an ELISA "sandwich assay," a capture antibody is immobilized on a solid surface of a microtiter plate to bind with a target antigen of a fluid sample. After washing the unbound material, an enzyme labeled antibody is bound to the same target antigen to detect the captured antigen. The binding of the enzyme labeled antibody to the target antigen is quantified by measuring the activity of the enzyme (e.g., by optical colorimetric or fluorescence reaction).

ELISAs can have different detection strategies, each with its drawbacks. Colorimetric detection is based on colorimetric reagents to form a soluble, colored product that accumulates over time, relative to the amount of enzyme present in each well. When the desired color intensity is reached, the product absorbance is measured. However, colorimetric detection has a lower sensitivity compared to fluorescent (e.g. fluorescence catalyzed by an enzyme)or chemiluminescent detection strategies.

Chemiluminescence detection is based on a chemical reaction that generates energy released in the form of light. Light emission occurs only during the enzyme-reagent reaction (e.g. enzyme-reagent reaction); therefore when the reagent becomes exhausted, the signal ceases. Therefore, chemiluminescent detection for ELISA can have poor signal intensity stability. For assays requiring many plates to be read, this can present a problem if the signal begins to decay before plates are read.

Fluorescent detection requires a fluorometer that produces the correct excitation beam to cause signal emission to be generated from the fluorescent tag. Thus, appropriate excitation and emission filters are needed.

These ELISAs detection techniques have further different drawbacks, including a training process for the operators, a slow measurement process as the operator needs to pipette fluid samples and calibration concentrations into each functionalized well. Additionally, ELISAs have long incubation times, use large volumes of reagents, have bulky dimensions and slow response time, which hinders the portable application of ELISAs. Furthermore, portable applications are limited due to their high cost compared to traditional ELISAs.

Examples of the present disclosure seek to at least partially reduce one or more of the aforementioned problems.

### SUMMARY

In a first aspect, a microfluidic device is provided. The microfluidic device comprises a substrate comprising one or more inlet channels; a mixing channel; wherein at least one inlet channel is fluidically connected to the mixing channel; a first chamber fluidically connected to the mixing channel; a capture antibody immobilized on the substrate in the first chamber; a second chamber fluidically connected to the first chamber and/or one or more inlet channels, wherein the second chamber comprises two or more electrodes; an outlet channel fluidically connected to the second chamber.

The microfluidic device further comprises a light source to irradiate the two or more electrodes of the second chamber.

According to this first aspect, a microfluidic device for an enzyme linked immunosorbent assay including a substrate and a light source is provided. The substrate may comprise a chemically functionalized surface suitable for immobilizing the capture antibody through physisorption (e.g. physical adsorption) or chemisorption (e.g. direct covalent bond). The chemically functionalized surface of the substrate may be flexible. The chemically functionalized surface allows obtaining the activation of the surface and the inherent introduction of reactive groups such as acids, amines, thiols, epoxy or alkenes. To obtain a chemically functionalized surface, reactive groups (e.g. acids, thiols, epoxy, amine, or alkenes) may be introduced to the substrate by a previous chlorosulfonation process and/or plasma surface treatment. The reactive groups allow strong anchoring of the capture antibodies to the surface of the substrate while preserving the activity of the capture antibodies and proper orientation of the capture antibodies. Therefore, the functionalization of the surface of the substrate allows selective and/or oriented immobilization of the capture antibodies depending on the reactive groups (e.g. acids, amines, thiols, epoxy or alkenes). According to this aspect, the sensitivity and/or detection limit of the microfluidic device for an enzyme linked immunosorbent assay may be increased by proper orientation and immobilization of the capture antibody.

In some examples, the substrate may be: aminated polystyrene, carboxylated polystyrene, thiolated polystyrene, epoxidized polystyrene, alkenylated polystyrene or chlorosulfonated polystyrene; the substrate having been obtained by functionalizing the surface of a polystyrene substrate. Non-functionalized polystyrene is very hydrophobic and not suitable for the interaction with biological agents (e.g. a capture antibody). Modifying the surface of the polystyrene by adding hydrophilic groups (e.g. acids, amine, thiols, epoxy or alkenes) may increase the wettability of the surface of the polystyrene and therefore may increase the hydrophilic properties and suitability of the polystyrene for biochemical application. Polystyrene functionalization may be done by a wet chemical treatment with chlorosulfonic acid and posterior washing with sulfuric acid. The wet chemical treatment with chlorosulfonic acid may provide reactive chlorosulfonyl groups on the surface of the polystyrene substrate. This functionalization may be used for subsequent introduction of any functional group by the interaction between a primary aliphatic amine or any hydrophilic group, and the chlorosulfonyl groups (creating sulphonamide groups when the chlorosulfonyl groups on the surface of the polystyrene substrate are contacted with a primary aliphatic amine) under mild conditions (e.g. aqueous solutions, room temperature). Moreover, a double functional spacer may be introduced, the spacer may allow decreasing the steric interactions between antibodies, maintaining their activity. Thus, functionalization of the polystyrene surface may increase the detection limit, and/or sensitivity. Therefore, the chemically functionalized flexible surface (e.g. an aminated polystyrene substrate, carboxylated polystyrene, thiolated polystyrene, epoxidized polystyrene, alkenylated polystyrene or chlorosulfonated polystyrene) may allow the proper orientation and/or the immobilization of the capture antibodies through chemisorption or physisorption. The immobilization and proper orientation of the capture antibody may be advantageous for high performance of an enzyme-linked immunosorbent assay through the facilitation of interactions between the capture antibody and the target antigen. The chemically functionalized surface of the substrate (e.g. aminated polystyrene, carboxylated polystyrene, thiolated polystyrene, epoxidized polystyrene, alkenylated polystyrene or chlorosulfonated polystyrene) may be flexible.

In some examples, the microfluidic device may further comprise a pressure sensor in each of the one or more inlet channels and the outlet channel; means to control the fluid flow through at least one of: the mixing channel, the first chamber and/or the second chamber. Therefore, the fluid sample, the enzyme labeled antibody may not be pipetted into functionalized inlets and no trained operators may be needed for conducting an ELISA.

In some examples, the microfluidic device further comprises at least one of the two or more electrodes comprising a surface with a conductive polymer layer. The conductive layer may facilitate the electron transfer from a semiconductor nano particle (e.g. cadmium sulfide) to the electrode surface. Conductive polymer layers may be used for cadmium sulfide immobilization while interacting with the electrode surface. Most frequently used are polypyrrole, polythiol, polyaniline and PEDOT polymers. In this example, conductive polymer layer may be an osmium polymer layer.

In some examples, the second chamber of the microfluidic device may further comprise: a conductive polymer layer; a gate electrically coupled to the conductive polymer layer; wherein the two or more electrodes are source and drain electrodes and, the source and the drain electrodes are separated from each other by the conductive polymer layer. Moreover, the conductive polymer layer may be an osmium polymer layer. Under illumination of a light source (e.g.an LED), a semiconductor nano particle (e.g. cadmium sulfide) may induce a gate voltage upon contact with the gate. Therefore, an ion transfer may occur between the electrolyte and the conductive polymer layer, which may change the doping state of the conductive polymer layer. Thus, small voltage signals applied to the gate may transduce into large changes in the drain current, allowing the electrochemical detection.

In some examples, the microfluidic device may be used for an enzyme linked immunosorbent assay, wherein at least a fluid sample, an enzyme labeled antibody, one or more reagents and cadmium ions or a compound that in solution yields cadmium ions are introduced in the one or more inlet channels; wherein a target antigen of the fluid sample is contacted with the enzyme labeled antibody to form a detection complex in the mixing channel; wherein the capture antibody immobilized on the substrate in the first chamber binds to the detection complex. As a result, the enzyme of the detection complex catalyzes: a reaction for obtaining a sulfur compound when the enzyme is contacted with the one or more reagents, cadmium sulfide nano particles being obtained when the sulfur compound is contacted with the cadmium ions; or alternately a reaction for stabilizing the generation of cadmium sulfide nano particles when the enzyme is contacted with the one or more reagents. Furthermore, the generated cadmium sulfide nano particles reach the surface of the two or more electrodes of the second chamber; and the light source irradiates the cadmium sulfide nano particles on the surface of the two or more electrodes to generate a signal representative of the presence of the target antigen. Therefore, an amplification of the electrochemical signal may be obtained by the enzymatic growth of cadmium nano particles. Sensibility and/or detection limit and/or detection time of the microfluidic device to detect a target antigen of a fluid sample may be improved in comparison with an ELISA kit.

In some examples, the microfluidic device, wherein the microfluidic device further comprises a control module comprising a reader module device, may further be used for an enzyme linked immunosorbent assay, wherein the reader module is configured to: receive the generated signal from the light source irradiating the cadmium sulfide nano particles on the surface of two or more electrodes; obtain a numerical value related to the generated signal; compare the numerical value to a predetermined threshold; determine the presence of the target antigen of the fluid sample depending on the comparison.

A microfluidic device may be a lab-on-a-chip, which is a miniaturized device that integrates one or several laboratory functions on a single compact platform. The microfluidic device or lab-on-a-chip may have a size from 1 square millimeter to 200 square centimeters in size, preferably between 100 square millimeters to 100 square centimeters. As a result, the microfluidic devices or lab-on a chip automatizes standard and routine laboratory procedures and conducts chemical and biochemical analysis in a miniaturized device. Miniaturization into a microfluidic device reduces volumes, dimensions and response time achieving better sensibilities and allows for the detection of different analytes in the same platform. Thus, the microfluidic devices or lab-on-a-chip may present a high throughput, cost-efficiency, high sensitivity, lack of cross-contamination and multi-detection of target antigen of a fluid sample. Moreover, microfluidic devices or lab-on-a-chip facilitate the use of reduced volumes, minimize the manipulation of dangerous materials and allow low reagent and low fluid sample consumption as well as less waste generation.

This way, a device that is simple, relatively fast, cost-effective, versatile with improved detection is provided.

According to a second aspect, a method to detect the presence of an antigen of a fluid sample using an enzyme-linked immunosorbent assay, the method comprising: providing a microfluidic device comprising a substrate with a mixing channel, a first chamber, a second chamber with two or more electrodes, wherein the two or more electrodes comprise a surface; contacting a target antigen of a fluid sample with an enzyme labeled antibody in the mixing channel to form a detection complex; contacting a capture antibody immobilized on the substrate in the first chamber with the detection complex; introducing one or more reagents into the first chamber such that the enzyme of the detection complex that is bound to the capture antibody immobilized on the substrate in the first chamber catalyzes: a reaction for obtaining a sulfur compound when contacted with the one or more reagents; or alternately a reaction for stabilizing the generation of cadmium sulfide nano particles when contacted with the one or more reagents; introducing cadmium ions or a compound that in solution yields cadmium ions into the first chamber to combine with the sulfur compound or with the one or more reagents and obtain cadmium sulfide nano particles; introducing thioglycerol into the second chamber; irradiating the cadmium sulfide nano particles on the surface of the two or more electrodes of the second chamber such as the thioglycerol acts as a redox mediator generating an electron flow through the cadmium sulfide nano particles to a conductive polymer layer and at least one electrode of the two or more electrodes of the second chamber, such as to generate a signal representative of the presence of the target antigen.

According to this second aspect, a method to detect the presence of an antigen of a fluid sample using an enzyme-linked immunosorbent assay is provided. The method may further comprise obtaining a numerical value related to the generated signal; comparing the numerical value related to the generated signal; determining the presence of the target antigen of the fluid depending on the comparison.

A fluid sample as used herein may be a blood sample, sample of a biological material, and/or a biological sample, wherein the fluid sample may contain a target antigen to detect.

A detection complex as used herein may comprise a target antigen of a fluid sample and an enzyme labeled antibody, wherein the enzyme labeled antibody may comprise a detectable enzyme label which is specific for (i.e., binds, is bound by, or forms a complex with) one or more reagents. Examples of detectable enzyme labels include enzyme labels such as, but not limited to, alkaline phosphatase (ALP), horseradish peroxidase (HRP), glucose oxidase, acetyl choline esterase (AChE) and glutathione reductase (GR).

A capture antibody may include an immobilized capture antibody which is specific for (i.e., binds, is bound by, or forms a complex with) one or more detection complex in an enzyme linked immunosorbent assay.

A reagent as used herein may be any substrate for an enzyme or a compound for an intermediate compound, wherein the intermediate compound may be produced when contacting an enzyme with one or more reagents.

A sulfur compound may be any compound containing sulfur suitable for obtaining cadmium sulfide nanoparticles when the sulfur compound is contacted with cadmium ions or a compound that in solution yields cadmium ions. In some examples, the sulfur compound may comprise sulfide ions, or hydrogen sulfide.

The catalyzed reaction on the substrate in the first chamber may be depending on the reagents and/or the enzyme of the detection complex. Therefore, the catalyzed reaction on the substrate in the first chamber may be for obtaining a sulfur compound when contacted with the one or more reagents; wherein the enzyme of the detection complex may be alkaline phosphatase (ALP) and the one or more reagents may comprise a thiol containing reagent such as sodium thiophosphate, wherein alkaline phosphatase (ALP) may catalyze the hydrolysis of the thiol containing reagent into a sulfur compound. Alternately, the enzyme of the detection complex may be horseradish peroxidase (HRP) and the one or more reagents may comprise sodium thiosulfate and hydrogen peroxide, wherein horseradish peroxidase (HRP) may catalyze the oxidation of sodium thiosulfate into a sulfur compound when contacted with hydrogen peroxide. Alternately, the enzyme of the detection complex may be glucose oxidase and the one or more reagents may comprise 1-thio-β-D-glucose and water H₂O, wherein glucose oxidase may catalyze the oxidation of 1-thio-β-D-glucose into 1-thio-β-D-gluconic acid, wherein 1-thio-β-D-gluconic acid may be hydrolyzed to β-D-gluconic acid and a sulfur compound when contacted with water H₂O. Alternately, the enzyme of the detection complex may be acetyl choline esterase (AChE) and the one or more reagents may comprise alkaline phosphatase (ALP) and thiosulfate, wherein acetyl choline esterase (AChE) may hydrolyze alkaline phosphatase (ALP) into thiocholine, wherein thiocholine may catalyze the hydrolysis of thiosulfate into a sulfur compound. Alternately, the catalyzed reaction on the substrate in the first chamber may be for stabilizing the generation of cadmium sulfide nano particles when contacted with the one or more reagents; wherein the enzyme of the detection complex may be glutathione reductase (GR) and the one or more reagents may comprise glutathione disulfide (GSSG) and NADPH, wherein glutathione reductase (GR) may catalyze the reduction of glutathione disulfide (GSSG) into glutathione (GSH) when contacted with NADPH, wherein the glutathione (GSH) may stabilizes the generation of cadmium sulfide nano particles; wherein the cadmium nano particles may be obtained by contacting cadmium ions or a compound that in solution yields cadmium ions with a sulfur compound.

Advantages derived from this second aspect may be similar to those mentioned regarding the microfluidic device of the first aspect. Namely, improved detection limit and detection time in comparison with ELISA kits.

In yet a further aspect, a kit comprising a microfluidic device and an enzyme labeled antibody, cadmium ions and one or more reagents to be introduced in the one or more inlet channels. As a result, the enzyme catalyzes a reaction for obtaining a sulfur compound when contacted with the one and more reagents; or alternately a reaction for stabilizing the generation of cadmium sulfide nano particles when contacted with the one or more reagents. Then, an ELISA test may thus be performed without a trained operator and/or a laboratory. The microfluidic device of this kit may be according to any of the examples herein disclosed.

### BRIEF DESCRIPTION OF THE DRAWINGS

Non-limiting examples of the present disclosure will be described in the following, with reference to the appended drawings, in which:
Figure 1a and 1-b illustrates a schematic diagram of a microfluidic device for an enzyme linked immunosorbent assay with a second chamber along the line A - A' or the line B - B', according to the present disclosure;
Figure 2a respectively shows a cross-sectional view of an example of the electrodes of a microfluidic device according to the present disclosure;
Figure 2b respectively shows a cross-sectional view of an example of the electrodes of a microfluidic device according to the present disclosure;

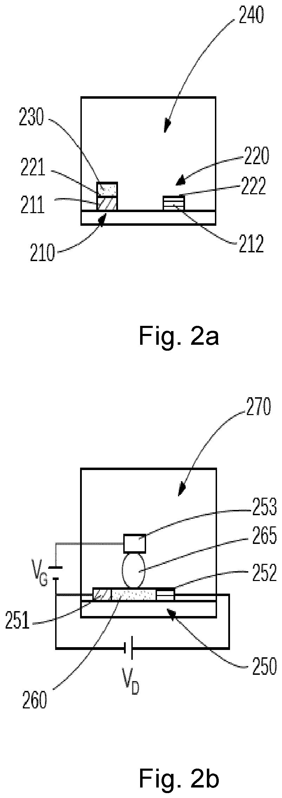

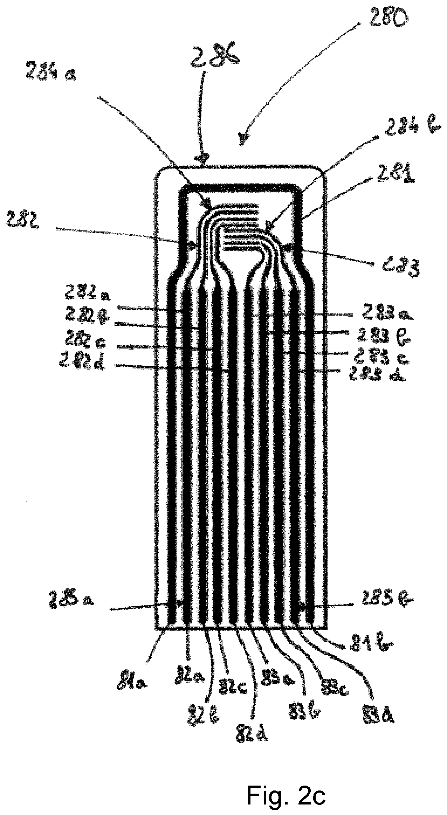

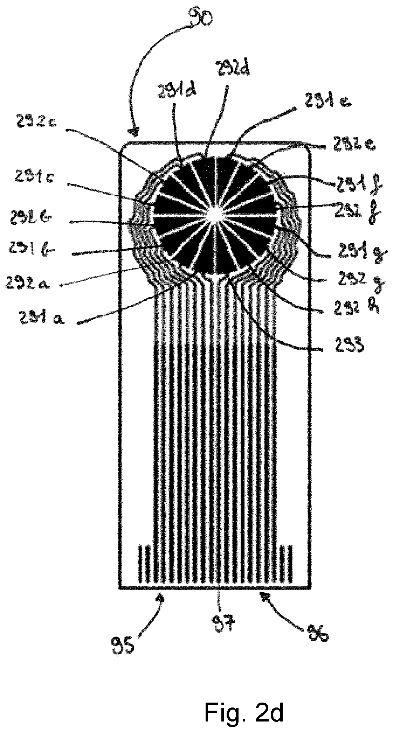

Figure 2 c respectively shows a top view of an examples of the electrodes of a microfluidic device according to the present disclosure;
Figure 2d respectively shows a top view of an example of the electrodes of a microfluidic device according to the present disclosure;

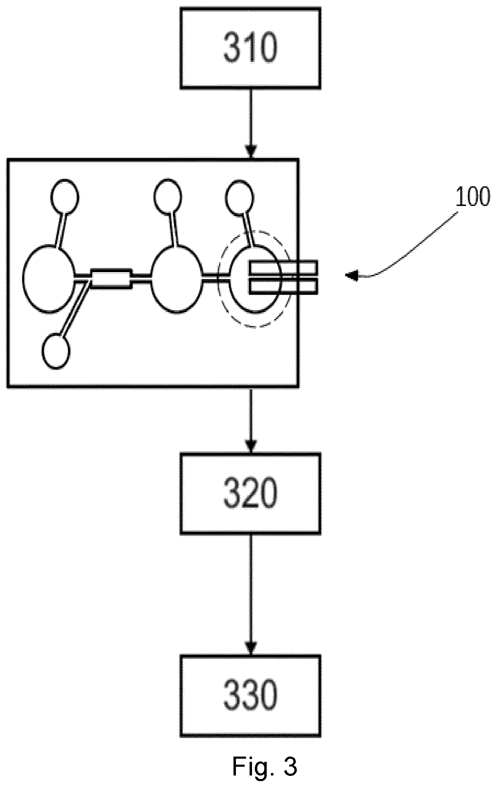

Figure 3 illustrates a block diagram of a device for detecting the presence of an antigen of a fluid sample using an enzyme-linked immunosorbent assay, according to the present disclosure;

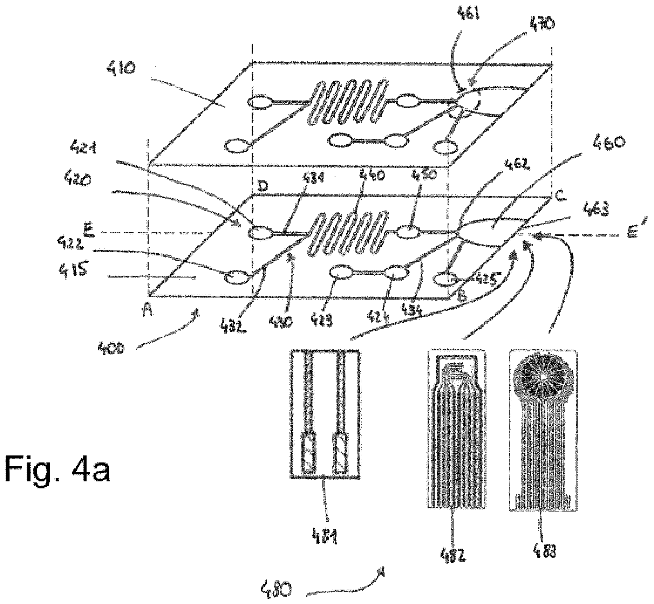

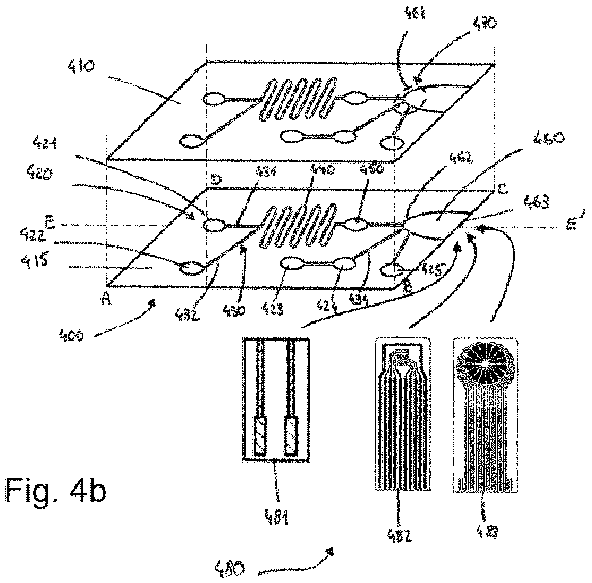

Figure 4a and 4b respectively show a microfluidic device with a second chamber along the line E - E' or the line F - F', according to some examples;

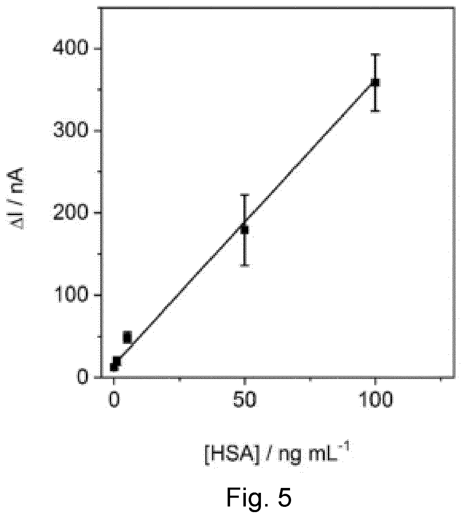

Figure 5 illustrates the maximum current obtained when a microfluidic device construed with an alkaline phosphatase enzyme labeled antibody, according to some examples;

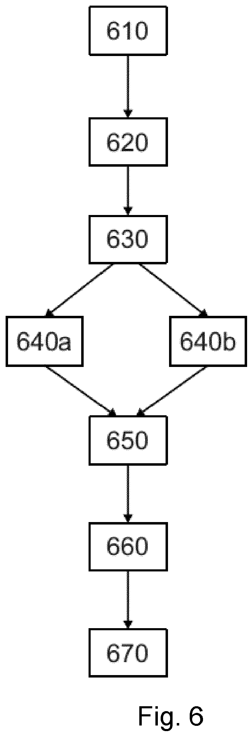

Figure 6 illustrates a flow chart of a method for detecting the presence of an antigen of a fluid sample using an enzyme-linked immunosorbent assay, according to an example.

### DETAILED DESCRIPTION OF EXAMPLES

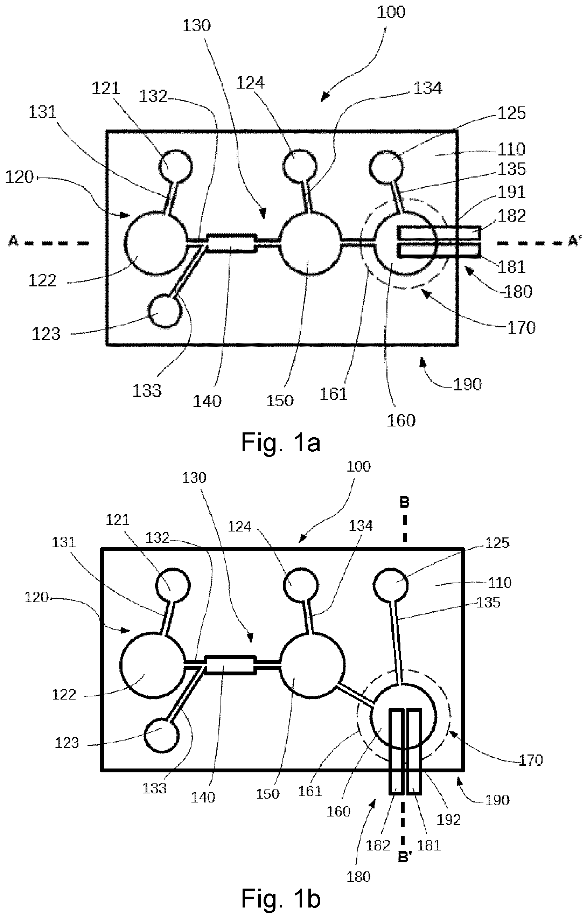

As can be seen in Figure 1, a microfluidic device 100 may comprise a substrate 110 comprising one or more inlet channels 120, a plurality of microfluidic channels 130, a mixing channel 140, a first chamber 150, a second chamber 160 and an outlet channel 170. At least one inlet channel 121 may be fluidically connected to at least one inlet channel 122 via a microfluidic channel 131. The inlet channel 122 may be fluidically connected to the mixing channel 140 via a microfluidic channel 132. The inlet channel 123 may be fluidically connected to the mixing channel 140 via a microfluidic channel 133. The mixing channel 140 may be fluidically connected to at least one of the one or more inlet channels 120 via the plurality of microfluidic channels 130. The first chamber 150 may be fluidically connected to the mixing channel 140 via a plurality of microfluidic channels 130. In the first chamber 150, a capture antibody may be immobilized on the substrate 100. The inlet channel 124 may be fluidically connected to the first chamber 150 via a microfluidic channel 134. The second chamber 160 may be fluidically connected to the first chamber 150 and /or one or more inlet channels 125. The second chamber 160 may comprise two or more electrodes 180. The two or more electrodes 180 may extend along a first longitudinal direction AA' or a second longitudinal direction BB'. The two or more electrodes 180 may at least partially extend in the second chamber 160 along a first longitudinal direction AA' or a second longitudinal direction BB'. The two or more electrodes 180 may at least partially extend outwardly from the second chamber 160 to an outer edge 190 of the microfluidic device 100. The two or more electrodes 180 may at least partially extend outwardly along a first longitudinal direction AA' from the second chamber 160 to an outer edge 191 of the microfluidic device 100. The two or more electrodes 180 may at least partially extend outwardly along a first longitudinal direction BB' from the second chamber 160 to an outer edge 192 of the microfluidic device 100. The two or more electrodes 180 may have a surface. In a two-electrode configuration, the electrode 181 may be the working electrode and the electrode 182 may be the counter electrode. The outlet channel 170 may be fluidically connected to the second chamber 160 via a plurality of microfluidic channels (not shown). Alternately, the second chamber 160 may have a top open end 161, wherein the top open end 161 of the second chamber 160 is the outlet channel 170.

In some examples, the two or more electrodes 180 may comprise carbon-based materials. Exemplary carbon-based materials are carbon cloth, carbon screen printed electrodes, carbon nanotubes.

In some examples, the two or more electrodes 180 may comprise gold, platinum, iron, nickel, copper, silver, silver chloride, carbon, screen printed electrodes, stainless steel, , tungsten and other metals suitable for electrode construction.

In this example, the working electrode 181 may comprise a carbon-based ink and/or the counter electrode 182 may comprise a silver-based ink.

In some examples, microfluidic devices, also referred to as lab-on-a-chip, may have any suitable structure. Such systems may be fabricated as a unitary structure from a single component, or as a multicomponent structure of two or more components. The two or more components may have any suitable relative spatial relationship and may be attached to one another by any suitable bonding mechanism such as a pressure sensitive adhesive.

In some examples, the chambers (e.g. reservoirs) may have a fluid capacity that is relatively large compared to the fluid capacity of the fluid network, so that they are not quickly exhausted. For example, the fluid capacity may be between 1 and 100 µL, more preferably between 2 and 50 µL.

In some examples, the microfluidic channels 130 may have a size between 1 and 2000µm, preferably between 1 and 1000µm more preferably between 100 and 500 µm.

In some examples, the microfluidic device 100 may include any other suitable means that contribute to fluid flow or flow analysis (not shown). For example, the microfluidic device 100 may include means to control fluid pressure and/or fluid flow rate (e.g. a pressure sensor) in each of the one or more inlet channels 120 and the outlet channel 170 and/or means to control the fluid flow through at least one of: the mixing channel 140, the first chamber 150 and/or the second chamber 160. Means to control the fluid flow or flow analysis may be pressure sensors, valves, and/or pumps (not shown). Means to control the fluid flow or flow analysis may operate by actively promoting flow and/or by restricting active or passive flow.

In some examples, the substrate 110 may be any suitable material or combination of suitable materials. Suitable materials may include elastomers, such as polydimethylsiloxane (PDMS); plastics (e.g. polystyrene, polypropylene, polycarbonate, cyclic olefin copolymer COC, cyclic olefin polymer COP, polymethyl methacrylate PMMA, polyethylene terephthalate PET), glass, ceramics; sol-gels; silicon and/or other metalloids, and biological polymers.

In some examples, the substrate 110 may comprise a chemically functionalized surface suitable for immobilizing the capture antibody through physisorption or chemisorption on the substrate 110 in the first chamber 150. The chemically functionalized surface allows obtaining the activation of the surface and inherent introduction of reactive groups such as acids, amines, thiols, epoxy or alkenes. To obtain a chemically functionalized flexible surface, reactive groups (e.g. acids, amine, thiols, epoxy or alkenes) may be introduced to the substrate 110 by a previous chlorosulfonation process and/or plasma surface treatment. The reactive groups allow strong anchoring of the capture antibodies to the surface of the substrate while preserving the activity of the capture antibodies and proper orientation of the capture antibodies. Therefore, the functionalization of the surface of the substrate allows selective and/or oriented immobilization of the capture antibodies depending on the reactive groups (e.g. acids, amines, or alkenes). The chemically functionalized surface of the substrate 110 may be flexible. The benefits of using a chemically functionalized flexible surface may be a higher sensitivity and/or detection limit of the microfluidic device for an enzyme linked immunosorbent assay by proper orientation and immobilization of the capture antibody.

In some examples, the substrate 110 may be aminated polystyrene, carboxylated polystyrene, thiolated polystyrene, epoxidized polystyrene, alkenylated polystyrene or chlorosulfonated polystyrene, the substrate 110 having been obtained by functionalizing the surface of a polystyrene substrate. Non-functionalized polystyrene is very hydrophobic and not suitable for the interaction with biological agents (e.g. a capture antibody). Modifying the surface of the polystyrene by adding hydrophilic groups (e.g. acids, amine, thiols, epoxy or alkenes) may increase the wettability of the surface of the polystyrene and therefore may increase the hydrophilic properties and suitability of the polystyrene for biochemical application. Polystyrene functionalization may be done by a wet chemical treatment with chlorosulfonic acid and posterior washing with sulfuric acid. The wet chemical treatment with chlorosulfonic acid may provide reactive chlorosulfonyl groups on the surface of the polystyrene substrate. This functionalization may be used for subsequent introduction of any functional group by the interaction between a primary aliphatic amine or any hydrophilic group, and the chlorosulfonyl groups (creating sulphonamide groups when the chlorosulfonyl groups on the surface of the polystyrene substrate are contacted with a primary aliphatic amine) under mild conditions (e.g. aqueous solutions, room temperature). In addition, a double functional spacer may be introduced, the spacer may allow decreasing the steric interactions between antibodies, maintaining their activity. Thus, the benefits of the functionalization of the polystyrene surface may be an increase of the detection limit, and/or sensitivity. Therefore, the chemically functionalized surface (e.g. an aminated polystyrene substrate, carboxylated polystyrene, thiolated polystyrene, epoxidized polystyrene, alkenylated polystyrene or chlorosulfonated polystyrene) may allow the proper orientation and/or the immobilization of the capture antibodies through chemisorption or physisorption. The chemically functionalized surface of the substrate (e.g. carboxylated polystyrene, thiolated polystyrene, epoxidized polystyrene, alkenylated polystyrene or chlorosulfonated polystyrene) may be flexible. The immobilization and proper orientation of the capture antibody may be advantageous for high performance of an enzyme-linked immunosorbent assay through the facilitation of interactions between the capture antibody and the target antigen.

The components of the microfluidic device 100 may be fabricated by any suitable mechanism, based on the desired application for the device and on materials used in fabrication. For example, one or more components may be molded, stamped, and/or embossed using a suitable mold. Such a mold may be formed of any suitable material by micromachining, etching, soft lithography, material deposition, cutting, and/or punching. Alternatively, components of a microfluidic system may be fabricated without a mold by etching, micromachining, cutting, punching, 3D-printing, and/or material deposition.

In some examples, the mixing channel 160 may be a serpentine channel with one or more inlet channels 120.

The microfluidic device 100 may be used for an enzyme linked immunosorbent assay, wherein at least a fluid sample, an enzyme labeled antibody, one or more reagents and cadmium ions or a compound that in solution yields cadmium ions are introduced in the one or more inlet channels 120; wherein a target antigen of the fluid sample is contacted with the enzyme labeled antibody to form a detection complex in the mixing channel 140; wherein the capture antibody immobilized on the substrate 100 in the first chamber 150 binds to the detection complex, such that the enzyme of the detection complex catalyzes: a reaction for obtaining a sulfur compound when the enzyme is contacted with the one or more reagents, cadmium sulfide nano particles being obtained when the sulfur compound is contacted with the cadmium ions; or alternately a reaction for stabilizing the generation of cadmium sulfide nano particles when the enzyme is contacted with the one or more reagents; wherein the cadmium sulfide nano particles reach the surface of the two or more electrodes of the second chamber.

In some examples, a kit may comprise: a microfluidic device 100 as hereinbefore described, comprising a substrate 110:
comprising one or more inlet channels 120,
a mixing channel 140, wherein at least one inlet channel 122-123 is fluidically connected to the mixing channel 140;
a first chamber 150 fluidically connected to the mixing channel 140;
a capture antibody immobilized on the substrate 110 in the first chamber 150;
a second chamber 160 fluidically connected to the first chamber 150 and/or one or more inlet channels 125, wherein the second chamber 160 comprises two or more electrodes 180, and
an outlet channel 170 fluidically connected to the second chamber 160
   and:
   an enzyme labeled antibody, cadmium ions and one or more reagents to be introduced in the one or more inlet channels, wherein the enzyme catalyzes:
   a reaction for obtaining a sulfur compound when contacted with the one and more reagents; or alternately
   a reaction for stabilizing the generation of cadmium sulfide nano particles when contacted with the one or more reagents.

In some examples. a microfluidic device 100 may further comprise a third inlet channel fluidically connected upstream of the first chamber 150 for introducing a washing buffer to remove unbound antigens, antibodies and/or proteins.

In some examples. a microfluidic device 100 may further comprise a third inlet channel fluidically connected upstream of the first chamber 150 for introducing: a thiol containing reagent such as sodium thiophosphate; or alternately sodium thiosulfate and/or hydrogen peroxide, 1-thio-β-D-glucose and/or water H₂O; or alternately alkaline phosphatase (ALP) and/or thiosulfate; or alternately glutathione disulfide (GSSG) and/or NADPH.

In some examples. a microfluidic device 100 may further comprise a fourth inlet channel fluidically connected upstream of the first chamber 150 for introducing:
a thiol containing reagent such as sodium thiophosphate; or alternately
sodium thiosulfate and/or hydrogen peroxide; or alternately
1-thio-β-D-glucose and/or water H₂O; or alternately
alkaline phosphatase (ALP) and/or thiosulfate; or alternately
glutathione disulfide (GSSG) and/or NADPH.

In some examples. a microfluidic device 100 may further comprise a fifth inlet channel fluidically connected upstream of the first chamber 150 for introducing cadmium ions.

In some examples, a microfluidic device 100 may further comprise a waste chamber (not shown) fluidically connected downstream of the first chamber 150.

In some examples, a microfluidic device 100 may further comprise a sixth inlet channel fluidically connected downstream of the first chamber 150 and fluidically connected upstream of the second chamber 160 for introducing thioglycerol. The reducing agent thioglycerol may amplify the photocurrent response of cadmium sulfide nanoparticles. Thioglycerol acts as a redox mediator generating an electron flow through the cadmium sulfide nano particles to a conductive osmium polymer layer and at least one electrode of the two or more electrodes. Contact of the cadmium sulfide nanoparticles with thioglycerol may be due because cadmium sulfide nanoparticles have high affinity to the thiol functional group. Therefore, the light source illuminating the cadmium sulfide nanoparticles and the cadmium sulfide nanoparticles may photocatalyze the oxidation of thioglycerol, generating photocurrent as the readout signal.

Figure 2a shows a cross-sectional view of an example of the electrodes of a microfluidic device 100 according to the present disclosure. In this example, the microfluidic device 100 as hereinbefore described that may comprise two or more electrodes 210, may further comprise a working electrode 211 and a counter electrode 212. The working electrode 211 and the counter electrode may have a surface 220. The surface 221 of the working electrode 211 may have a conductive polymer layer 230. The conductive polymer layer may be an osmium polymer layer. The conductive layer may facilitate the electron transfer from a semiconductor nano particle (e.g. cadmium sulfide) in the second chamber 240 to the surface 221 of the working electrode 211.

In some examples, the electrodes 210 may comprise gold, platinum, iron, nickel, copper, silver, silver chloride, carbon, screen printed electrodes, stainless steel, , tungsten and other metals suitable for electrode Figure 2b shows a cross-sectional view of an example of the electrodes of a microfluidic device 100 according to the present disclosure. In this example, the microfluidic device 100 as hereinbefore described that may comprise two or more electrodes 250 in the second chamber 270, may further comprise three electrodes, namely a source electrode 251, a drain electrode 252 and a gate electrode 253. The source electrode 251 and the drain electrode 252 may be separated from each other by a conductive polymer layer 260. The conductive polymer layer may be an osmium polymer layer. The gate electrode 253 is electrically coupled to the conductive polymer layer. Source electrode 251 and drain electrode 252 may establish electrical contact to the conductive polymer layer 260, while the gate electrode 253 may establish electrical contact to electrolyte 265. Electrolyte 265 may be liquid, gel, or solid. In the most common configuration as shown in Figure 2b, source electrode 251 may be grounded and a voltage (drain voltage VD) may be applied to the drain. This may cause a current to flow (drain current), due to electronic charges (e.g. holes) present in the conductive polymer layer 260. When a voltage is applied to the gate (gate voltage VG), ions from the electrolyte 265 may be injected in the conductive polymer layer 260 and may change the electronic charge density, and hence the drain current. When the gate voltage is removed and the gate is shorted to the source, the injected ions may return to the electrolyte and the drain current may go back to its original value.

Figure 2c shows a top view of an example of the electrodes of a microfluidic device 100 according to the present disclosure. In this example, the two of more electrodes of the microfluidic device 100 as hereinbefore described in Figure 2c may be an organic electrochemical transistor 280. The organic electrochemical transistor 280 may comprise a first working electrode 281, a second working electrode 282 with a plurality of electrodes terminals 282a, 282b, 282c, 282d and a reference electrode 283 with a plurality of electrodes terminals 283a, 283b, 283c, 283d. The second working electrode 282 and the reference electrode 283 may be separated from each other by a conductive polymer layer 287. The conductive polymer layer may be an osmium polymer layer. The second working electrode may have a proximal part 284a and a distal part 285a. The reference electrode may have a proximal part 284b and a distal part 285b. The distal part 285a of the second working electrode 282 may have connection terminals 82a, 82b, 82c, 82d to connect the second working electrode 282 to a control module (not shown). The distal part 285b of the reference electrode 283 may have connection terminals 83a, 83b, 83c, 83d to connect the reference electrode 283 to a control module (not shown). The first working electrode 281 may have connection terminals 81a, 81b to connect the first electrode 281 to a control module (not shown). The organic electrochemical transistor 280 may be embedded in the second chamber 286 of a microfluidic device 100.

Figure 2d shows a top view of an example of the electrodes of a microfluidic device 100 according to the present disclosure. In this example, the microfluidic device 100 as hereinbefore described that may comprise two or more electrodes 290 in the second chamber 295, may further comprise three electrodes, namely a working electrode 291, a counter electrode 292 ad a reference electrode 293. The working electrode may have a plurality of electrodes 291a, 291b, 291c, 291d, 291e, 291f, 291g with a proximal end 296a and a distal end 297a. The proximal end 296a of the plurality of electrodes of the working electrode 291 may comprise a surface 298a, 298b, 298c, 298d, 298d, 298e, 298f, 298g. The counter electrode may have a plurality of electrodes 292a, 292b, 292c, 292d, 292e, 292f, 292g, 292 with a proximal end 296b and a distal end 297b. The proximal end 296b of the plurality of electrode terminals of the counter electrode 292 may comprise a surface 299a, 299b, 299c, 299d, 299d, 299e, 299f, 299g. The surface 298a, 298b, 298c, 298d, 298d, 298e, 298f, 298g of the working electrode 291 may have a conductive polymer layer 294. The conductive polymer layer 294 may be an osmium polymer layer. The conductive layer may facilitate the electron transfer from a semiconductor nano particle (e.g. cadmium sulfide) in the second chamber 295 to the surface 298a, 298b, 298c, 298d, 298d, 298e, 298f, 298g of the working electrode 291. The reference electrode 293 may have a proximal part 296c and a distal part 297c. The distal part 297a of the working electrode 291 may have connection terminals 95 to connect the working electrode 291 to a control module (not shown). The distal part 297b of the counter electrode 292 may have connection terminals 96 to connect the counter electrode 292 to a control module (not shown). The distal part 297c of the reference electrode 293 may have connection terminals 97 to connect the reference electrode 293 to a control module (not shown). The three electrodes (i.e. working electrode, counter electrode and reference electrode) may be embedded in the second chamber 90 of a microfluidic device 100.

In some examples, the electrodes 290 may comprise carbon-based materials. Exemplary carbon-based materials are carbon cloth, carbon screen printed electrodes, carbon nanotubes.

In some examples, the electrodes 290 may comprise gold, platinum, iron, nickel, copper, silver, silver chloride, carbon, screen printed electrodes, stainless steel, , tungsten and other metals suitable for electrode construction.

In this example, the working electrode 291 may comprise a carbon-based ink and/or the counter electrode 292 may comprise a silver-based ink.

In this example, the two or more electrodes 290, may further comprise three screen printed electrodes comprising a working electrode 291 based on carbon ink, a counter electrode 292 based on carbon ink and a reference 293 electrode based on silver ink.

In the Figure 2a, 2b, 2c and 2d only the second chamber 240, 270, 286, 90 of a microfluidic device 100 as hereinbefore described is shown. However, the microfluidic device may comprise a substrate comprising one or more inlet channels, a plurality of microfluidic channels, a mixing channel, a first chamber, a second chamber and an outlet channel as hereinbefore described, namely a source electrode 211, a drain electrode 252 and a gate electrode 253. The source electrode 251 and the drain electrode 252 may be separated from each other by a conductive polymer layer 260. The conductive polymer layer may be an osmium polymer layer.

Figure 3 illustrates a block diagram of a device to detect the presence of an antigen of a fluid sample using an enzyme-linked immunosorbent assay, according to some examples. A microfluidic device 100 as hereinbefore described in Figure 1, 2a, 2b, 2c, 2d, may further comprise a light source 310. The light source 310 may irradiate the two or more electrodes of the second chamber. The light source 310 may be an LED such as an UV-LED. The microfluidic device 100, may further comprise a reader module 320 configured to receive the generated signal from the light source 310 irradiating cadmium sulfide nano particles on the surface of the two or more electrodes.

The microfluidic device may further comprise a reader module 320 configured to receive one or more response signal from the light source that irradiates the cadmium sulfide nano particles on the surface of the two or more electrodes to generate a signal representative of the presence of the target antigen and to obtain one or more features of the response signal (e.g. the amperometry and/or the voltammetry and/or potentiometry). The system may further comprise a control module 330.

The control module 330 may comprise or may be implemented by electronic means, computing means or a combination of them, that is, the electronic or computing means may be used interchangeably so that a part of the described means may be electronic means and the other part may be computing means, or all described means may be electronic means or all described means may be computing means.

Examples of a control module 330 comprising only electronic means (that is, a purely electronic configuration) may be a programmable electronic device such as a CPLD (Complex Programmable Logic Device), an FPGA (Field Programmable Gate Array) or an ASIC (Application-Specific Integrated Circuit).

Examples of a control module 330 comprising only computing means may be a computer system (e.g. an embedded computer, etc.), which may comprise a memory and a processor, the memory being adapted to store a set of computer program instructions, and the processor being adapted to execute these instructions stored in the memory in order to generate the various events and actions for which the control module has been programmed.

The computer program may comprise program instructions for causing the control module 330 to perform a method to detect the presence of an antigen of a fluid sample using an enzyme-linked immunosorbent assay. The computer program may be embodied on a storage medium such as a ROM, for example a CD ROM or a semiconductor ROM, a magnetic recording medium, for example a hard disk, a solid-state disk (SSD), a USB flash drive (for example, a pen drive); or a non-volatile memory card such as a SD, miniSD or microSD card. In addition, the computer program may be carried on a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

The computer program may be in the form of source code, object code, a code intermediate source and object code such as in partially compiled form, or in any other form suitable for use in the implementation of the method. The carrier may be any entity or device capable of carrying the computer program.

Moreover, the control module 330 may also have a hybrid configuration between computing and electronic means. In this case, the control module may comprise a memory and a processor to implement computationally part of its functionalities and certain electronic circuits to implement the remaining functionalities.

The reader module 320 may be composed of electronics to transmit information between the control module and the information system. The information system: may store the necessary operations and commands to operate the whole reader system transmit one or more features of the response signal (e.g. the amperometry and/or the voltammetry and/or potentiometry).

In any case, the control module 330 may be configured to execute a method to detect the presence of an antigen of a fluid sample using an enzyme-linked immunosorbent assay, wherein the method comprises:
obtaining a numerical value related to the generated signal;
comparing the numerical value related to the generated signal;
determining the presence of the target antigen of the fluid depending on the comparison.

In some examples as hereinbefore described, the microfluidic device 100 may be used for an enzyme linked immunosorbent assay, wherein at least a fluid sample, an enzyme labeled antibody, one or more reagents and cadmium ions or a compound that in solution yields cadmium ions are introduced in the one or more inlet channels 120; wherein a target antigen of the fluid sample is contacted with the enzyme labeled antibody to form a detection complex in the mixing channel 140; wherein the capture antibody immobilized on the substrate 100 in the first chamber 150 binds to the detection complex, such that the enzyme of the detection complex catalyzes: a reaction for obtaining a sulfur compound when the enzyme is contacted with the one or more reagents, cadmium sulfide nano particles being obtained when the sulfur compound is contacted with the cadmium ions; or alternately a reaction for stabilizing the generation of cadmium sulfide nano particles when the enzyme is contacted with the one or more reagents; wherein the cadmium sulfide nano particles reach the surface of the two or more electrodes of the second chamber and wherein the light source 310 irradiates the cadmium sulfide nano particles on the surface of the two or more electrodes to generate a signal representative of the presence of the target antigen.

In some examples, a kit may comprise: a microfluidic device 100 as hereinbefore described, comprising a substrate 110 comprising:
one or more inlet channels 120,
a mixing channel 140, wherein at least one inlet channel 122-123 is fluidically connected to the mixing channel 140;
a first chamber 150 fluidically connected to the mixing channel 140;
a capture antibody immobilized on the substrate 110 in the first chamber 150;
a second chamber 160 fluidically connected to the first chamber 150 and/or one or more inlet channels 125, wherein the second chamber 160 comprises two or more electrodes 180,
an outlet channel 170 fluidically connected to the second chamber 160
   and:
   an enzyme labeled antibody, cadmium ions and one or more reagents to be introduced in the one or more inlet channels, wherein the enzyme catalyzes:
   a reaction for obtaining a sulfur compound when contacted with the one and more reagents; or alternately
   a reaction for stabilizing the generation of cadmium sulfide nano particles when contacted with the one or more reagents.

Figure 4 shows a microfluidic device with a second chamber along the line E - E' or the line F - F', according to some examples. As can be seen in Figure 4, a microfluidic device 400 may comprise a first substrate 410 as the top plate, a second substrate 415 as the bottom plate. The second substrate 415 may construe a micro-fluidic system of the enzyme -linked immunosorbent assay lab on-a-chip by the inner surfaces of the first substrate 410 comprising one or more inlet channels 420, a plurality of microfluidic channels 430, a mixing channel 440 that is a serpentine, a first chamber 450, a second chamber 460 and an outlet channel 470. The inlet channel 421 may be fluidically connected to the mixing channel 440 via a microfluidic channel 431. The inlet channel 422 may be fluidically connected to the mixing channel 440 via a microfluidic channel 432. The mixing channel 440 may be fluidically connected to at least one of the one or more inlet channels 420 via the plurality of microfluidic channels 430. The first chamber 450 may be fluidically connected to the mixing channel 440 via a plurality of microfluidic channels 430. In the first chamber 450, a capture antibody may be immobilized on the substrate 400. The inlet channel 424 may be fluidically connected to the second chamber 450 via a microfluidic channel 434. The inlet channel 425 may be fluidically connected to the second chamber 450 via a microfluidic channel 435. The second chamber 460 may be fluidically connected to the first chamber 450 and /or one or more inlet channels 423-425. The second chamber 460 may extend along a first longitudinal direction EE' or a second longitudinal direction FF' from a coupling end 462 to an open end 463. The coupling end 462 is fluidically connected to the first chamber 450. Instead of having a second chamber with two or more electrodes, the second chamber 460 may extend outwardly along a first direction EE' or a second direction FF'. Electrochemical sensors 481-483 having two or more electrode may have a proximal end with two or more electrodes and a distal end, where the distal end of the electrochemical sensors may comprise two or more electrode connection terminals. The two or more electrode connection terminals of the electrochemical sensors may connect the two or more electrodes to a control module (not shown). The electrochemical sensors 481-483 may be at least partially inserted into the open end 463 of the second chamber 460. The outlet channel 470 may be fluidically connected to the second chamber 460 via a plurality of microfluidic channels (not shown). Alternately, the second chamber 460 may have a top open end 461, wherein the top open end 461 of the second chamber 460 is the outlet channel 470. The bottom plate 415 is bonded to the top plate 410 to compose the micro-fluidic system of the enzyme-linked immunosorbent assay lab-on-a-chip such that at least one longitudinal direction of a first longitudinal direction along the axis A, B, C, D is substantially parallel to the second longitudinal direction along the axis A, B, C, D.

Electrochemical sensor 481 is analogous to the example of the electrodes of a microfluidic device according to the present disclosure of Figure 2a hereinbefore described. The two or more electrodes may be embedded in a sensor (e.g. an electrochemical sensor) substrate that may be at least partially inserted into the open end 463 of the second chamber 460 of the microfluidic device 400.

Electrochemical sensor 482 is analogous to the example of the electrodes of a microfluidic device according to the present disclosure of Figure 2b or Figure 2c, hereinbefore described. The two or more electrodes may be embedded in a sensor (e.g. an electrochemical sensor) substrate that may be at least partially inserted into the open end 463 of the second chamber 460 of the microfluidic device 400.

Electrochemical sensor 483 is analogous to the example of the electrodes of a microfluidic device according to the present disclosure of Figure 2d, hereinbefore described. The two or more electrodes may be embedded in a sensor (e.g. an electrochemical sensor) substrate that may be at least partially inserted into the open end 463 of the second chamber 460 of the microfluidic device 400.

In some examples, the electrode connections may be connected to a control module comprising a reader module by a carrier (e.g. transmissible carrier such as an electrical or optical signal, which may be conveyed via electrical or optical cable or by radio or other means). The reader module may be configured to receive one or more response signal from a light source that irradiates the cadmium sulfide nano particles on the surface of the two or more electrodes to generate a signal representative of the presence of the target antigen and to obtain one or more features of the response signal (e.g. the amperometry and/or the voltammetry and/or potentiometry).

The reader module may be composed of electronics to transmit information between the control module and the information system. The information system: may store the necessary operations and commands to operate the whole reader system transmit one or more features of the response signal (e.g. the amperometry and/or the voltammetry and/or potentiometry).

The system may further comprise a control module. The control module may comprise or may be implemented by electronic means, computing means or a combination of them, that is, the electronic or computing means may be used interchangeably so that a part of the described means may be electronic means and the other part may be computing means, or all described means may be electronic means or all described means may be computing means.

In any case, the control module may be configured to execute a method to detect the presence of an antigen of a fluid sample using an enzyme-linked immunosorbent assay, wherein the method comprises:
obtaining a numerical value related to the generated signal;
comparing the numerical value related to the generated signal;
determining the presence of the target antigen of the fluid depending on the comparison.

In some examples, microfluidic devices, also referred to as lab-on-a-chip, may have any suitable structure. Such systems may be fabricated as a unitary structure from a single component, or as a multicomponent structure of two or more components. The two or more components may have any suitable relative spatial relationship and may be attached to one another by any suitable bonding mechanism such as a pressure sensitive adhesive.

In some examples, two or more of the components may be fabricated as relatively thin layers, which may be disposed face-to-face. The relatively thin layers may have distinct thickness, based on function. For example, the thickness of some layers may be between 10 to 2500 µm, preferably 20 to 2000 µm, and more preferably 20 to 1000 µm, among others. Other layers may be substantially thicker, in some cases providing mechanical strength to the system. The thicknesses of such other layers may be about 0.25 to 2 cm, 0.4 to 1.5 cm, or 0.5 to 1 cm.

In some examples, microfluidic components may be fabricated separately, joined, and further modified as appropriate. For example, when fabricated as distinct layers, microfluidic components may be bonded, generally face-to-face.

The chambers (e.g. reservoirs) may have a fluid capacity that is relatively large compared to the fluid capacity of the fluid network, so that they are not quickly exhausted. For example, the fluid capacity may be between 1 and 300 µL, preferably 1 and 100 µL, more preferably between 2 and 50 µL,

In some examples, the microfluidic device 400 may include any other suitable means that contribute to fluid flow or flow analysis (not shown). For example, the microfluidic device 400 may include means to control fluid pressure and/or fluid flow rate (e.g. a pressure sensor) in each of the one or more inlet channels 420 and the outlet channel 470 and/or means to control the fluid flow through at least one of: the mixing channel 440, the first chamber 450 and/or the second chamber 460. Means to control the fluid flow or flow analysis may be pressure sensors, valves, and/or pumps (not shown). Means to control the fluid flow or flow analysis may operate by actively promoting flow and/or by restricting active or passive flow.

In some examples, the top substrate 410 or the bottom substrate 415 may be any suitable material or combination of suitable materials. Suitable materials may include elastomers, such as polydimethylsiloxane (PDMS); plastics (e.g. polystyrene, polypropylene, polycarbonate, cyclic olefin copolymer COC, cyclic olefin polymer COP, polymethyl methacrylate PMMA, polyethylene terephthalate PET), glass, ceramics; sol-gels; silicon and/or other metalloids, and biological polymers.

In some examples, the first substrate and the second substrate are substantially the same.

In some examples, the top substrate 410 or the bottom substrate 415 may comprise a chemically functionalized surface suitable for immobilizing the capture antibody through physisorption or chemisorption on the top substrate 410 or the bottom substrate 415 in the first chamber 450. The chemically functionalized surface allows obtaining the activation of the surface and inherent introduction of reactive groups such as acids, amines, thiols, epoxy or alkenes. To obtain a chemically functionalized surface, reactive groups (e.g. acids, amine, thiols, epoxy or alkenes) may be introduced to the top substrate 410 or the bottom substrate 415 by a previous chlorosulfonation process and/or plasma surface treatment. The reactive groups allow strong anchoring of the capture antibodies to the surface of the substrate while preserving the activity of the capture antibodies and proper orientation of the capture antibodies. Therefore, the functionalization of the surface of the substrate allows selective and/or oriented immobilization of the capture antibodies depending on the reactive groups (e.g. acids, amines, thiols, epoxy or alkenes). The chemically functionalized surface of the substrate 410, 415 may be flexible. The benefits of using a chemically functionalized flexible surface may be a higher sensitivity and/or detection limit of the microfluidic device for an enzyme linked immunosorbent assay by proper orientation and immobilization of the capture antibody.

In some examples, the top substrate 410 or the bottom substrate 415 may be aminated polystyrene, carboxylated polystyrene, thiolated polystyrene, epoxidized polystyrene, alkenylated polystyrene or chlorosulfonated polystyrene, the top substrate 410 or the bottom substrate 415 having been obtained by functionalizing the surface of a polystyrene substrate. Non-functionalized polystyrene is very hydrophobic and not suitable for the interaction with biological agents (e.g. a capture antibody). Modifying the surface of the polystyrene by adding hydrophilic groups (e.g. acids, amine, thiols, epoxy or alkenes) may increase the wettability of the surface of the polystyrene and therefore may increase the hydrophilic properties and suitability of the polystyrene for biochemical application. Polystyrene functionalization may be done by a wet chemical treatment with chlorosulfonic acid and posterior washing with sulfuric acid. The wet chemical treatment with chlorosulfonic acid may provide reactive chlorosulfonyl groups on the surface of the polystyrene substrate. This functionalization may be used for subsequent introduction of any functional group by the interaction between a primary aliphatic amine or any hydrophilic group, and the chlorosulfonyl groups (creating sulphonamide groups when the chlorosulfonyl groups on the surface of the polystyrene substrate are contacted with a primary aliphatic amine) under mild conditions (e.g. aqueous solutions, room temperature). In addition, a double functional spacer may be introduced, the spacer may allow decreasing the steric interactions between antibodies, maintaining their activity. Thus, the benefits of the functionalization of the polystyrene surface may be an increase of the detection limit, and/or sensitivity. Therefore, the chemically functionalized flexible surface (e.g. an aminated polystyrene, carboxylated polystyrene, thiolated polystyrene, epoxidized polystyrene, alkenylated polystyrene or chlorosulfonated polystyrene substrate) may allow the proper orientation and/or the immobilization of the capture antibodies through chemisorption or physisorption. The chemically functionalized surface of the substrate (e.g. carboxylated polystyrene, thiolated polystyrene, epoxidized polystyrene, alkenylated polystyrene or chlorosulfonated polystyrene) may be flexible. The immobilization and proper orientation of the capture antibody may be advantageous for high performance of an enzyme-linked immunosorbent assay through the facilitation of interactions between the capture antibody and the target antigen.

The components of the microfluidic device 400 may be fabricated by any suitable mechanism, based on the desired application for the device and on materials used in fabrication. For example, one or more components may be molded, stamped, and/or embossed using a suitable mold. Such a mold may be formed of any suitable material by micromachining, etching, soft lithography, material deposition, cutting, and/or punching. Alternatively, components of a microfluidic system may be fabricated without a mold by etching, micromachining, cutting, punching, 3D-printing, and/or material deposition.

In some examples, the microfluidic channels 430 may have a size between 1 and 2000µm, preferably between 1 and 1000µm more preferably between 100 and 500 µm.

In some examples, the mixing channel 460 may be a serpentine channel with one or more inlet channels 420.

The microfluidic device 400 may be used for an enzyme linked immunosorbent assay, wherein at least a fluid sample, an enzyme labeled antibody, one or more reagents and cadmium ions or a compound that in solution yields cadmium ions are introduced in the one or more inlet channels 420; wherein a target antigen of the fluid sample is contacted with the enzyme labeled antibody to form a detection complex in the mixing channel 440; wherein the capture antibody immobilized on the substrate 415 in the first chamber 450 binds to the detection complex, such that the enzyme of the detection complex catalyzes: a reaction for obtaining a sulfur compound when the enzyme is contacted with the one or more reagents, cadmium sulfide nano particles being obtained when the sulfur compound is contacted with the cadmium ions; or alternately a reaction for stabilizing the generation of cadmium sulfide nano particles when the enzyme is contacted with the one or more reagents; wherein the cadmium sulfide nano particles reach the surface of the two or more electrodes of the second chamber.

In some examples, a kit may comprise: a microfluidic device 400 as hereinbefore described, comprising a substrate 415
comprising one or more inlet channels 420,
a mixing channel 440, wherein at least one inlet channel 421-422 is fluidically connected to the mixing channel 440;
a first chamber 450 fluidically connected to the mixing channel 440;
a capture antibody immobilized on the substrate 415 in the first chamber 450;
a second chamber 460 fluidically connected to the first chamber 450 and/or one or more inlet channels 423-425, wherein the second chamber 460 comprises an electrochemical sensor 481-483 with two or more electrodes, and
an outlet channel 470 fluidically connected to the second chamber 460
a light source to irradiate the two or more electrodes of the second chamber
   and:
   an enzyme labeled antibody, cadmium ions and one or more reagents to be introduced in the one or more inlet channels, wherein the enzyme catalyzes:
   a reaction for obtaining a sulfur compound when contacted with the one and more reagents; or alternately
   a reaction for stabilizing the generation of cadmium sulfide nano particles when contacted with the one or more reagents.

In some examples. a microfluidic device further comprising a third inlet channel fluidically connected upstream of the first chamber for introducing a washing buffer to remove unbound antigens, antibodies and/or proteins.

In some examples. a microfluidic device 400 may further comprise a third inlet channel fluidically connected upstream of the first chamber 450 for introducing: a thiol containing reagent such as sodium thiophosphate; or alternately sodium thiosulfate and/or hydrogen peroxide, 1-thio-β-D-glucose and/or water H₂O; or alternately alkaline phosphatase (ALP) and/or thiosulfate; or alternately glutathione disulfide (GSSG) and/or NADPH.

In some examples. a microfluidic device 400 may further comprise a fourth inlet channel fluidically connected upstream of the first chamber 450 for introducing:
a thiol containing reagent such as sodium thiophosphate; or alternately
sodium thiosulfate and/or hydrogen peroxide; or alternately
1-thio-β-D-glucose and/or water H₂O; or alternately
alkaline phosphatase (ALP) and/or thiosulfate; or alternately
glutathione disulfide (GSSG) and/or NADPH.

In some examples, a microfluidic device 400 may further comprise further comprising a fifth inlet channel fluidically connected upstream of the first chamber 450 for introducing cadmium ions.

In some examples, a microfluidic device 400 may further comprise a waste chamber fluidically connected downstream of the first chamber 450.

In some examples, a microfluidic device 400 may further comprise a sixth inlet channel fluidically connected downstream of the first chamber 450 and fluidically connected upstream of the second chamber 460 for introducing thioglycerol. The reducing agent thioglycerol may amplify the photocurrent response of cadmium sulfide nanoparticles. Thioglycerol acts as a redox mediator generating an electron flow through the cadmium sulfide nano particles to a conductive osmium polymer layer and at least one electrode of the two or more electrodes. Contact of the cadmium sulfide nanoparticles with thioglycerol may be due because cadmium sulfide nanoparticles have high affinity to the thiol functional group. Therefore, the light source illuminating the cadmium sulfide nanoparticles and the cadmium sulfide nanoparticles may photocatalyze the oxidation of thioglycerol, generating photocurrent as the readout signal.

Figure 5, It may be seen that photoelectrochemical signal increases gradually with Human serum albumin HSA concentrations from 0 to 100 ng mL-1 due to the increased enzymatic growth of cadmium nanoparticles. The curve may exhibit a linear response in the range studied. The detection limit may be comparable to a commercial that may not have the benefits of a faster time response (e.g. 2,5 hours).

Figure 6 is a flow chat schematically illustrating a method 600 to detect the presence of an antigen of a fluid sample using an enzyme linked immunosorbent assay, according to an example. Such detection method is performable by/through devices similar to the one described with reference to Figure 1

Firstly, in block 610, a microfluidic device comprising a substrate with a mixing channel, a first chamber, a second chamber with two or more electrodes that may comprise a surface, may be provided.

In block 620, a target antigen of a fluid sample may be contacted with an enzyme labeled antibody in the mixing channel to form a detection complex.

In block 630, a capture antibody immobilized on the substrate in the first chamber may be contacted with the detection complex.

In block 640a, one or more reagents may be introduced into the first chamber such that the enzyme of the detection complex that is bound to the capture antibody immobilized on the substrate of the first chamber catalyzes a reaction for obtaining a sulfur compound when contacted with the one or more reagents.

Alternately, in block 640b one or more reagents may be introduced into the first chamber such that the enzyme of the detection complex that is bound to the capture antibody immobilized on the substrate of the first chamber catalyzes a reaction for stabilizing the generation of cadmium sulfide nano particles when contacted with the one or more reagents.

The catalyzed reaction on the substrate in the first chamber may be depending on the reagents and/or the enzyme of the detection complex. Therefore, the catalyzed reaction on the substrate in the first chamber may be for obtaining a sulfur compound when contacted with the one or more reagents or for stabilizing the generation of cadmium sulfide nano particles when contacted with the one or more reagents.

In block 640b, a sulfur compound to contact with cadmium ions or a compound that yields cadmium in solution to form cadmium sulfide nanoparticles may be introduced in one or more inlet channels such that the catalyzed reaction may stabilize the generation of cadmium sulfide nanoparticles.

In block 650, cadmium ions or a compound that in solution yields cadmium ions may be introduced into the first chamber to combine with the sulfur compound or with the one or more reagents and obtain cadmium sulfide nano particles.

In block 660, thioglycerol may be introduced into the second chamber.

In block 670, the cadmium sulfide nano particles on the surface of the two or more electrodes of the second chamber may be irradiated such as the thioglycerol acts as a redox mediator generating an electron flow through the cadmium sulfide nano particles to a conductive polymer layer and at least one electrode of the two or more electrodes of the second chamber, such as to generate a signal representative of the presence of the target antigen.

A method 600 further comprising obtaining a numerical value related to the generated signal; comparing the numerical value related to the generated signal; determining the presence of the target antigen of the fluid depending on the comparison.

In an example, the enzyme of the enzyme labeled antibody may comprise alkaline phosphatase (ALP) or horseradish peroxidase (HRP) or glucose oxidase or acetyl choline esterase (AChE) or glutathione reductase (GR).

In an example, the enzyme of the detection complex may comprise alkaline phosphatase (ALP) and the one or more reagents may comprise a thiol containing reagent such as sodium thiophosphate. In this example, alkaline phosphatase (ALP) may catalyze the hydrolysis of the thiol containing reagent into a sulfur compound that may be used in method 600 in the block 650.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. A microfluidic device comprising:
a substrate comprising:
one or more inlet channels;
a mixing channel; wherein at least one inlet channel is fluidically connected to the mixing channel;
a first chamber fluidically connected to the mixing channel;
a capture antibody immobilized on the substrate in the first chamber;
a second chamber fluidically connected to the first chamber and/or one or more inlet channels, wherein the second chamber comprises two or more electrodes, wherein at least one of the two or more electrodes comprises a surface with a conductive polymer layer;
an outlet channel fluidically connected to the second chamber;
a light source to irradiate the two or more electrodes of the second chamber.

2. A device according to claim 1, wherein the substrate comprises at least one of the following: aminated polystyrene substrate, carboxylated polystyrene substrate, thiolated polystyrene substrate, epoxidized polystyrene substrate, alkenylated polystyrene substrate, chlorosulfonated polystyrene substrate.

3. A device according to any of claims 1 to 2, further comprising a control module comprising a reader module.

4. A device according to any of claims 1 to 3, the second chamber further comprising:
a conductive polymer layer;
a gate electrically coupled to the conductive polymer layer;
wherein the two or more electrodes are source and drain electrodes and, the source and the drain electrodes are separated from each other by the conductive polymer layer.

5. A device according to claim 3 or 4, wherein the conductive polymer layer is an osmium polymer layer.

6. A device according to any of claims 1 to 5, further comprising:
a pressure sensor in each of the one or more inlet channels and the outlet channel;
means to control the fluid flow through at least one of: the mixing channel, the first chamber and/or the second chamber.

7. A device according to any of claims 1 to 6, wherein the light source is an LED.

8. The use of a microfluidic device according to any of claims 1 to 7 for an enzyme linked immunosorbent assay:
wherein at least a fluid sample, an enzyme labeled antibody, one or more reagents and cadmium ions or a compound that in solution yields cadmium ions are introduced in the one or more inlet channels;
wherein a target antigen of the fluid sample is contacted with the enzyme labeled antibody to form a detection complex in the mixing channel;
wherein the capture antibody immobilized on the substrate in the first chamber binds to the detection complex, such that the enzyme of the detection complex catalyzes:
a reaction for obtaining a sulfur compound when the enzyme is contacted with the one or more reagents, cadmium sulfide nano particles being obtained when the sulfur compound is contacted with the cadmium ions; or alternately
a reaction for stabilizing the generation of cadmium sulfide nano particles when the enzyme is contacted with the one or more reagents;
wherein the cadmium sulfide nano particles reach the surface of the two or more electrodes of the second chamber;
and wherein the light source irradiates the cadmium sulfide nano particles on the surface of the two or more electrodes to generate a signal representative of the presence of the target antigen.

9. The use of claim 8, wherein the microfluidic device is according to any of claims 4 to 7 and the reader module is configured to:
receive the generated signal from the light source irradiating the cadmium sulfide nano particles on the surface of two or more electrodes;
obtain a numerical value related to the generated signal;
compare the numerical value to a predetermined threshold;
determine the presence of the target antigen of the fluid sample depending on the comparison.

10. A kit comprising:
a microfluidic device according to any of claims 1 to 7;
and:
an enzyme labeled antibody, cadmium ions and one or more reagents to be introduced in the one or more inlet channels,
wherein the enzyme catalyzes:
a reaction for obtaining a sulfur compound when contacted with the one and more reagents, cadmium sulfide nano particles being obtained when the sulfur compound is contacted with the cadmium ions; or alternately
a reaction for stabilizing the generation of cadmium sulfide nano particles when contacted with the one or more reagents.

11. A method to detect the presence of an antigen of a fluid sample using an enzyme-linked immunosorbent assay, the method comprising:
providing a microfluidic device comprising a substrate with a mixing channel, a first chamber, a second chamber with two or more electrodes, wherein the two or more electrodes comprise a surface;
contacting a target antigen of a fluid sample with an enzyme labeled antibody in the mixing channel to form a detection complex;
contacting a capture antibody immobilized on the substrate in the first chamber with the detection complex;
introducing one or more reagents into the first chamber such that the enzyme of the detection complex that is bound to the capture antibody immobilized on the substrate in the first chamber catalyzes:
a reaction for obtaining a sulfur compound when contacted with the one or more reagents; or alternately
a reaction for stabilizing the generation of cadmium sulfide nano particles when contacted with the one or more reagents;
introducing cadmium ions or a compound that in solution yields cadmium ions into the first chamber to combine with the sulfur compound or with the one or more reagents and obtain cadmium sulfide nano particles;
introducing thioglycerol into the second chamber;
irradiating the cadmium sulfide nano particles on the surface of the two or more electrodes of the second chamber such as the thioglycerol acts as a redox mediator generating an electron flow through the cadmium sulfide nano particles to a conductive polymer layer and at least one electrode of the two or more electrodes of the second chamber, such as to generate a signal representative of the presence of the target antigen.

12. A method according to claim 11, the method further comprising:
obtaining a numerical value related to the generated signal;
comparing the numerical value related to the generated signal;
determining the presence of the target antigen of the fluid depending on the comparison.

13. A method according to any of claims 11 to 12, wherein the enzyme of the enzyme labeled antibody is alkaline phosphatase (ALP) or horseradish peroxidase (HRP) or glucose oxidase or acetyl choline esterase (AChE) or glutathione reductase (GR).

14. A method according to any of claims 11 to 12:
wherein the enzyme of the detection complex is alkaline phosphatase (ALP) and the one or more reagents comprise a thiol containing reagent such as sodium thiophosphate, wherein alkaline phosphatase (ALP) catalyzes the hydrolysis of the thiol containing reagent into a sulfur compound.

## Patentansprüche

1. Eine mikrofluidische Vorrichtung, umfassend:
ein Substrat, umfassend:
einen oder mehrere Einlasskanäle;
einen Mischkanal; wobei mindestens ein Einlasskanal fluidisch mit dem Mischkanal verbunden ist;
eine erste Kammer, die fluidisch mit dem Mischkanal verbunden ist;
einen Einfangantikörper, der auf dem Substrat in der ersten Kammer immobilisiert ist;
eine zweite Kammer, die fluidisch mit der ersten Kammer und/oder einem oder mehreren Einlasskanälen verbunden ist, wobei die zweite Kammer zwei oder mehr Elektroden umfasst, wobei mindestens eine der zwei oder mehr Elektroden eine Oberfläche mit einer leitfähigen Polymerschicht umfasst;
einen Auslasskanal, der fluidisch mit der zweiten Kammer verbunden ist;
eine Lichtquelle, um die zwei oder mehr Elektroden der zweiten Kammer zu bestrahlen.

2. Eine Vorrichtung nach Anspruch 1, wobei das Substrat mindestens eines der folgenden umfasst: aminiertes Polystyrolsubstrat, carboxyliertes Polystyrolsubstrat, thioliertes Polystyrolsubstrat, epoxidiertes Polystyrolsubstrat, alkenyliertes Polystyrolsubstrat, chlorsulfoniertes Polystyrolsubstrat.

3. Eine Vorrichtung nach einem der Ansprüche 1 bis 2, ferner umfassend ein Steuermodul, das ein Lesemodul umfasst.

4. Eine Vorrichtung nach einem der Ansprüche 1 bis 3, wobei die zweite Kammer ferner Folgendes umfasst:
eine leitfähige Polymerschicht;
ein Gate, das elektrisch an die leitfähige Polymerschicht gekoppelt ist;
wobei die zwei oder mehr Elektroden Source- und Drain-Elektroden sind und die Source- und die Drain-Elektroden durch die leitfähige Polymerschicht voneinander getrennt sind.

5. Eine Vorrichtung nach Anspruch 3 oder 4, wobei die leitfähige Polymerschicht eine Osmiumpolymerschicht ist.

6. Eine Vorrichtung nach einem der Ansprüche 1 bis 5, ferner umfassend:
einen Drucksensor in jedem von dem einen oder den mehreren Einlasskanälen und dem Auslasskanal;
ein Mittel zum Steuern des Fluidstroms durch mindestens eines von: dem Mischkanal, der ersten Kammer und/oder der zweiten Kammer.

7. Eine Vorrichtung nach einem der Ansprüche 1 bis 6, wobei die Lichtquelle eine LED ist.

8. Die Verwendung einer mikrofluidischen Vorrichtung nach einem der Ansprüche 1 bis 7 für einen Enzym-gebundenen Immunosorbent-Assay:
wobei mindestens eine flüssige Probe, ein enzymmarkierter Antikörper, ein oder mehrere Reagenzien und Cadmiumionen oder eine Verbindung, die in Lösung Cadmiumionen liefert, in den einen oder die mehreren Einlasskanäle eingeführt werden;
wobei ein Zielantigen der flüssigen Probe mit dem enzymmarkierten Antikörper in Kontakt gebracht wird, um einen Detektionskomplex in dem Mischkanal zu bilden;
wobei der auf dem Substrat in der ersten Kammer immobilisierte Einfangantikörper an den Detektionskomplex bindet, so dass das Enzym des Detektionskomplexes Folgendes katalysiert:
eine Reaktion zur Gewinnung einer Schwefelverbindung, wenn das Enzym mit dem einen oder den mehreren Reagenzien in Kontakt gebracht wird, wobei Cadmiumsulfid-Nanopartikel erhalten werden, wenn die Schwefelverbindung mit den Cadmiumionen in Kontakt gebracht wird; oder alternativ
eine Reaktion zum Stabilisieren der Erzeugung von Cadmiumsulfid-Nanopartikeln, wenn das Enzym mit dem einen oder den mehreren Reagenzien in Kontakt gebracht wird;
wobei die Cadmiumsulfid-Nanopartikel die Oberfläche der zwei oder mehr Elektroden der zweiten Kammer erreichen;
und wobei die Lichtquelle die Cadmiumsulfid-Nanopartikel auf der Oberfläche der zwei oder mehr Elektroden bestrahlt, um ein Signal zu erzeugen, das für die Anwesenheit des Zielantigens repräsentativ ist.

9. Die Verwendung von Anspruch 8, wobei die mikrofluidische Vorrichtung nach einem der Ansprüche 4 bis 7 ist und das Lesemodul konfiguriert ist, um:
das erzeugte Signal von der Lichtquelle zu empfangen, die die Cadmiumsulfid-Nanopartikel auf der Oberfläche von zwei oder mehr Elektroden bestrahlt;
einen numerischen Wert in Bezug auf das erzeugte Signal zu erhalten;
den Zahlenwert mit einem vorbestimmten Schwellenwert zu vergleichen;
das Vorhandensein des Zielantigens der Flüssigkeitsprobe in Abhängigkeit von dem Vergleich zu bestimmen.

10. Ein Kit umfassend:
eine mikrofluidische Vorrichtung nach einem der Ansprüche 1 bis 7;
und:
einen enzymmarkierten Antikörper, Cadmiumionen und ein oder mehrere Reagenzien, die in den einen oder die mehreren Einlasskanäle eingeführt werden sollen,
wobei das Enzym Folgendes katalysiert:
eine Reaktion zur Gewinnung einer Schwefelverbindung bei Kontakt mit dem einen und den mehreren Reagenzien, wobei Cadmiumsulfid-Nanopartikel erhalten werden, wenn die Schwefelverbindung mit den Cadmiumionen in Kontakt gebracht wird; oder alternativ
eine Reaktion zur Stabilisierung der Erzeugung von Cadmiumsulfid-Nanopartikeln, wenn sie mit dem einen oder den mehreren Reagenzien in Kontakt gebracht werden.

11. Ein Verfahren zum Nachweisen des Vorhandenseins eines Antigens einer flüssigen Probe unter Verwendung eines *enzyme-linked Immunosorbent Assays,* wobei das Verfahren Folgendes umfasst:
bereitstellen einer mikrofluidischen Vorrichtung, die ein Substrat mit einem Mischkanal, einer ersten Kammer, einer zweiten Kammer mit zwei oder mehr Elektroden umfasst, wobei die zwei oder mehr Elektroden eine Oberfläche umfassen;
in-Kontakt-bringen eines Zielantigens einer flüssigen Probe mit einem enzymmarkierten Antikörper in dem Mischkanal, um einen Detektionskomplex zu bilden;
in-Kontakt-bringen eines auf dem Substrat immobilisierten Einfangantikörpers in der ersten Kammer mit dem Detektionskomplex;
einbringen eines oder mehrerer Reagenzien in die erste Kammer, so dass das Enzym des Detektionskomplexes, das an den auf dem Substrat in der ersten Kammer immobilisierten Einfangantikörper gebunden ist, Folgendes katalysiert:
eine Reaktion zur Gewinnung einer Schwefelverbindung bei Kontakt mit dem einen oder den mehreren Reagenzien; oder alternativ
eine Reaktion zur Stabilisierung der Erzeugung von Cadmiumsulfid-Nanopartikeln, wenn sie mit dem einen oder den mehreren Reagenzien in Kontakt gebracht werden;
einführen von Cadmiumionen oder einer Verbindung, die in Lösung Cadmiumionen liefert, in die erste Kammer, um sich mit der Schwefelverbindung oder mit dem einen oder den mehreren Reagenzien zu verbinden und Cadmiumsulfid-Nanopartikel zu erhalten;
einführen von Thioglycerin in die zweite Kammer;
bestrahlen der Cadmiumsulfid-Nanopartikel auf der Oberfläche der zwei oder mehr Elektroden der zweiten Kammer, so dass das Thioglycerin als ein Redoxvermittler dient, der einen Elektronenstrom durch die Cadmiumsulfid-Nanopartikel zu einer leitfähigen Polymerschicht und mindestens einer Elektrode der zwei oder mehr Elektroden der zweiten Kammer erzeugt, um ein Signal zu erzeugen, das für die Anwesenheit des Zielantigens repräsentativ ist.

12. Ein Verfahren nach Anspruch 11, wobei das Verfahren ferner Folgendes umfasst:
erhalten eines auf das erzeugte Signal bezogenen numerischen Werts;
vergleichen des auf das erzeugte Signal bezogenen numerischen Werts;
bestimmen des Vorhandenseins des Zielantigens der Flüssigkeit in Abhängigkeit von dem Vergleich.

13. Ein Verfahren nach einem der Ansprüche 11 bis 12, wobei das Enzym des enzymmarkierten Antikörpers alkalische Phosphatase (ALP) oder Meerrettichperoxidase (HRP) oder Glucoseoxidase oder Acetylcholinesterase (AChE) oder Glutathionreduktase (GR) ist.

14. Ein Verfahren nach einem der Ansprüche 11 bis 12:
wobei das Enzym des Nachweiskomplexes alkalische Phosphatase (ALP) ist und das eine oder die mehreren Reagenzien ein thiolhaltiges Reagenz wie Natriumthiophosphat umfassen, wobei die alkalische Phosphatase (ALP) die Hydrolyse des thiolhaltigen Reagenz in eine Schwefelverbindung katalysiert.

## Revendications

1. Un dispositif microfluidique comprenant :
un substrat comprenant :
un ou plusieurs canaux d'entrée ;
un canal de mélange ; dans lequel au moins un canal d'entrée est connecté de manière fluidique au canal de mélange ;
une première chambre reliée fluidiquement au canal de mélange ;
un anticorps de capture immobilisé sur le substrat dans la première chambre ;
une seconde chambre reliée de manière fluidique à la première chambre et/ou à un ou plusieurs canaux d'entrée, dans lequel la seconde chambre comprend deux ou plusieurs électrodes, dans lequel au moins l'une des deux ou plusieurs électrodes comprend une surface avec une couche de polymère conducteur ;
un canal de sortie connecté fluidiquement à la seconde chambre ;
une source de lumière pour irradier les deux ou plusieurs électrodes de la seconde chambre.

2. Un dispositif selon la revendication 1, dans lequel le substrat comprend au moins l'un des éléments suivants : un substrat de polystyrène aminé, un substrat de polystyrène carboxylé, un substrat de polystyrène thiolaté, un substrat de polystyrène époxydé, un substrat de polystyrène alcénylaté, un substrat de polystyrène chlorosulfonaté.

3. Un dispositif selon l'une quelconque des revendications 1 à 2, comprenant en outre un module de commande comprenant un module lecteur.

4. Un dispositif selon l'une quelconque des revendications 1 à 3, la seconde chambre comprenant en outre :
une couche de polymère conducteur ;
un portillon couplé électriquement à la couche de polymère conducteur ;
dans lequel les deux ou plusieurs électrodes sont des électrodes de source et de drain et les électrodes de source et de drain sont séparées l'une de l'autre par la couche de polymère conducteur.

5. Un dispositif selon la revendication 3 ou 4, dans lequel la couche de polymère conducteur est une couche de polymère d'osmium.

6. Un dispositif selon l'une quelconque des revendications 1 à 5, comprenant en outre :
un capteur de pression dans chacun des un ou plusieurs canaux d'entrée et le canal de sortie ;
un moyen pour contrôler l'écoulement de fluide à travers au moins l'un des éléments suivants : le canal de mélange, la première chambre et/ou la seconde chambre.

7. Un dispositif selon l'une quelconque des revendications 1 à 6, dans lequel la source de lumière est une DEL.

8. L'utilisation d'un dispositif microfluidique selon l'une quelconque des revendications 1 à 7 pour un essai d'immunoadsorption par enzyme liée :
dans laquelle au moins un échantillon de fluide, un anticorps marqué par une enzyme, un ou plusieurs réactifs et des ions de cadmium ou un composé qui, en solution, donne des ions de cadmium sont introduits dans le ou les canaux d'entrée ;
dans laquelle un antigène cible de l'échantillon de fluide est mis en contact avec l'anticorps marqué par une enzyme pour former un complexe de détection dans le canal de mélange ;
dans laquelle l'anticorps de capture immobilisé sur le substrat dans la première chambre se lie au complexe de détection, de sorte que l'enzyme du complexe de détection catalyse :
une réaction pour obtenir un composé de soufre lorsque l'enzyme est mise en contact avec l'un ou les plusieurs réactifs, des nanoparticules de sulfure de cadmium étant obtenues lorsque le composé de soufre est mis en contact avec les ions cadmium ; ou alternativement
une réaction pour stabiliser la génération de nanoparticules de sulfure de cadmium lorsque l'enzyme est mise en contact avec l'un ou les plusieurs réactifs ;
dans laquelle les nanoparticules de sulfure de cadmium atteignent la surface des deux ou plusieurs électrodes de la seconde chambre ;
et dans laquelle la source de lumière irradie les nano particules de sulfure de cadmium sur la surface des deux ou plusieurs électrodes pour générer un signal représentatif de la présence de l'antigène cible.

9. L'utilisation de la revendication 8, dans laquelle le dispositif microfluidique est selon l'une quelconque des revendications 4 à 7 et le module lecteur est configuré pour :
recevoir le signal généré par la source de lumière irradiant les nanoparticules de sulfure de cadmium sur la surface de deux ou plusieurs électrodes ;
obtenir une valeur numérique liée au signal généré ;
comparer la valeur numérique à un seuil prédéterminé ;
déterminer la présence de l'antigène cible de l'échantillon de fluide en fonction de la comparaison.

10. Un kit comprenant :
un dispositif microfluidique selon l'une quelconque des revendications 1 à 7 ;
et :
un anticorps marqué par une enzyme, des ions cadmium et un ou plusieurs réactifs à introduire dans l'un ou les plusieurs canaux d'entrée,
dans lequel l'enzyme catalyse :
une réaction pour obtenir un composé de soufre lorsqu'elle est mise en contact avec l'un ou les plusieurs réactifs, des nanoparticules de sulfure de cadmium étant obtenues lorsque le composé de soufre est mis en contact avec les ions de cadmium ; ou alternativement
une réaction pour stabiliser la génération de nanoparticules de sulfure de cadmium lorsqu'elles sont mises en contact avec l'un ou les plusieurs réactifs.

11. Un procédé pour détecter la présence d'un antigène d'un échantillon de fluide en utilisant un dosage immuno-absorbant lié à une enzyme, le procédé comprenant :
fournir un dispositif microfluidique comprenant un substrat avec un canal de mélange, une première chambre, une seconde chambre avec deux ou plusieurs électrodes, dans lequel les deux ou plusieurs électrodes comprennent une surface ;
mettre en contact un antigène cible d'un échantillon de fluide avec un anticorps marqué par une enzyme dans le canal de mélange pour former un complexe de détection ;
mettre en contact un anticorps de capture immobilisé sur le substrat dans la première chambre avec le complexe de détection ;
introduire un ou plusieurs réactifs dans la première chambre de sorte que l'enzyme du complexe de détection qui est liée à l'anticorps de capture immobilisé sur le substrat dans la première chambre catalyse :
une réaction pour obtenir un composé de soufre lorsqu'elle est mise en contact avec l'un ou les plusieurs réactifs ; ou alternativement
une réaction pour stabiliser la génération de nanoparticules de sulfure de cadmium lorsqu'elles sont mises en contact avec l'un ou les plusieurs réactifs ;
l'introduction d'ions de cadmium ou d'un composé qui, en solution, donne des ions de cadmium dans la première chambre pour se combiner avec le composé de soufre ou avec l'un ou les plusieurs réactifs et obtenir des nanoparticules de sulfure de cadmium ;
l'introduction de thioglycérol dans la seconde chambre ;
l'irradiation des nanoparticules de sulfure de cadmium sur la surface des deux ou plusieurs électrodes de la seconde chambre de sorte que le thioglycérol agit comme un médiateur redox générant un flux d'électrons à travers les nanoparticules de sulfure de cadmium vers une couche de polymère conducteur et au moins une électrode des deux ou plusieurs électrodes de la seconde chambre, de manière à générer un signal représentatif de la présence de l'antigène cible.

12. Un procédé selon la revendication 11, le procédé comprenant en outre :
obtenir une valeur numérique liée au signal généré ;
comparer la valeur numérique liée au signal généré ;
déterminer la présence de l'antigène cible du fluide en fonction de la comparaison.

13. Un procédé selon l'une quelconque des revendications 11 à 12, dans lequel l'enzyme de l'anticorps marqué par l'enzyme est la phosphatase alcaline (ALP) ou la peroxydase de raifort (HRP) ou la glucose oxydase ou l'acétylcholinestérase (AChE) ou la glutathion réductase (GR).

14. Un procédé selon l'une quelconque des revendications 11 à 12 :
dans lequel l'enzyme du complexe de détection est la phosphatase alcaline (ALP) et l'un ou les plusieurs réactifs comprennent un réactif contenant du thiol tel que le thiophosphate de sodium, dans lequel la phosphatase alcaline (ALP) catalyse l'hydrolyse du réactif contenant du thiol pour donner un composé de soufre.
